# EUROPEAN PATENT SPECIFICATION

(11) **EP 3 407 883 B1**
(45) Date of publication and mention of the grant of the patent: **25.08.2021**
(21) Application number: 17711314.9
(22) Date of filing: 25.01.2017
(51) Int. Cl.: A61K 31/365, A61K 31/405, A61K 33/06, A61K 33/08, A61K 36/28, A61K 36/48, A61P 25/06

(54) **FORMULATION FOR TREATING MIGRAINE**
FORMULIERUNG ZUR BEHANDLUNG VON MIGRÄNE
FORMULATION POUR TRAITER LA MIGRAINE

(30) Priority: 29.01.2016 IT UB20160516
(43) Date of publication of application: 05.12.2018
(73) Proprietor: GOTHAM S.r.l., 25122 Brescia (IT)
(72) Inventor: DALLA VOLTA, Giorgio, 25122 Brescia (IT)
(74) Representative: Long, Giorgio
(86) International application number: PCT/IB2017/050378
(87) International publication number: WO 2017/130108

(56) References cited:
- WO-A1-99/23881
- US-A1- 2004 086 582
- US-A1- 2005 186 269
- US-A1- 2006 233 892
- US-A1- 2010 284 986
- US-B1- 6 500 450
- Anonymous: "Hedec Cronic 500 di GLAUBER PHARMA (60 compresse)", , 1 May 2015 (2015-05-01), XP055310075, Retrieved from the Internet: URL:https://web.archive.org/web/2015050113 5058/http://www.iafstore.com/ita/glauber-p harma/hedec-cronic-500-codp28355 [retrieved on 2016-10-12]
- TORI HUDSON: "Relieving Migraine Headaches in Women", ALTERNATIVE AND COMPLEMENTARY THERAPIES, vol. 13, no. 1, 1 February 2007 (2007-02-01), pages 36-38, XP055310189, US ISSN: 1076-2809, DOI: 10.1089/act.2006.13101
- NANCY E. KELLEY ET AL: "Rescue Therapy for Acute Migraine, Part 1: Triptans, Dihydroergotamine, and Magnesium", HEADACHE., vol. 52, no. 1, 28 December 2011 (2011-12-28), pages 114-128, XP055373927, United States ISSN: 0017-8748, DOI: 10.1111/j.1526-4610.2011.02062.x
- P ZAVARISE ET AL: "A Combination of Tanacetum parthenium, Griffonia simplicifolia and Magnesium (Aurastop) as Symptomatic Acute Treatment for Migraine Aura: A Retrospective Cohort Study", OPEN ACCESS LIBRARY JOURNAL, vol. 4, no. e3660, 8 June 2017 (2017-06-08), pages 1-9, XP055723286, ISSN: 2333-9705, DOI: 10.4236/oalib.1103660
- GIORGIO DALLA VOLTA ET AL: "Comparison of the Effect of Tanacethum Parthenium, 5-Hydroxy Tryptophan, and Magnesium (Aurastop) versus Magnesium Alone on Aura Phenomenon and Its Evolution", PAIN RESEARCH AND MANAGEMENT, vol. 2019, 9 October 2019 (2019-10-09), pages 1-4, XP055723117, US ISSN: 1203-6765, DOI: 10.1155/2019/6320163
- Anonymous: "Hedec Cronic 500 - Glauber Pharma", , 7 January 2015 (2015-01-07), pages 1-3, XP055785245, Retrieved from the Internet: URL:https://www.iafstore.com/ita/glauber-p harma/hedec-cronic-500codp28555 [retrieved on 2021-03-12]
- Della Volta Giorgio: "Impact of Aurastop on the aura phenomena - Conference paper - Association of Tanacethum Parthenium, 5-hydroxy-tryptophan and magnesium impact on aura evolution: unexpected results.", , 30 November 2016 (2016-11-30), pages 1-5, XP055785229, Retrieved from the Internet: URL:https://www.researchgate.net/publicati on/311534364_Association_of_tanachetum_par thenium_5HTP_magnesium_impact_on_aura_evol ution_unexpected_result [retrieved on 2021-03-12]
- Anonymous: "Product AURASTOP. A new association of components that is able to block migraine aura.", , 4 February 2021 (2021-02-04), pages 1-6, XP055785201, Retrieved from the Internet: URL:https://aurastop.it/sanitary-operator- area [retrieved on 2021-03-12]

## Description

The present invention relates to a formulation for use in the treatment of migraine with aura.

Migraine is a frequent and debilitating headache, as shown by various epidemiological studies and is ranked by the World Health Organization in nineteenth place (twelfth for women) in the classification of disabling diseases.

The current classification of the International Headache Society, the International Classification of Headache Disorders (ICHD-III, 2013) distinguishes two major subtypes of migraine: migraine "without aura", a clinical syndrome characterized by headache with distinctive features and associated symptoms, and headache "with aura", characterized by focal neurological symptoms that usually precede, but sometimes accompany the pain phase.

The prevalence of migraine ranges from 15% to 18% in females, and from 6% to 12% in males. Hormonal factors play a major role in the increased prevalence of migraine in women. In a sample aged from 25 to 64 years old, the incidence of migraine was 8.1 per 1,000 people per year.

Symptomatic treatment alone of migraine is indicated when a debilitating headache is present for fewer than four days per month. Preventive therapy should instead be established alongside the symptomatic therapy, if a debilitating headache is present for at least four days a month.

The main objectives of a prophylactic therapy are to reduce the frequency of migraine attacks and the patient's disability, improving the quality of life, and restoring acceptable physical efficiency.

Before embarking on a prophylactic treatment it is a good idea to try to identify all the triggers and aggravating factors by keeping a diary and wherever possible eliminate them, which may in itself reduce the frequency and/or intensity of the attacks.

In prophylactic therapy medicines belonging to the pharmacological classes of beta-blockers, calcium-antagonists, serotonin antagonists, antidepressants and anti-epileptics are used.

The exact mechanisms of action of these medicines in preventing migraines is not known. An action on the vascular, neuronal and neurotransmitter alteration mechanisms underlying the pathogenesis of migraine is supposed.

Despite the existing symptomatic and prophylactic therapies, there is still a need to search for new combinations of active substances for the treatment of migraine, particularly in the light of the social impact it entails.

The web page of the product "HEDEC Cronic 500 di Glauber Pharma (60 compresse)" discloses a composition similar to the one of the present invention, but it does not describe the treatment of migraine with aura with such a composition.

WO 99/23881 A1 discloses the combined use of 5-HTP and magnesium for treating migraine with aura. However the composition described therein does not comprise *Tanacetum parthenium.*

US 2004/086582 A1 discloses the use of partenolide or feverfew extract for treating migraine with aura. However the composition described therein does not comprise 5-HTP and magnesium.

Nancy E.K. et al., Headache, vol. 52, no. 1, 28 December 2011, pp. 114-128 discloses that magnesium is effective to treat migraine with aura. However the composition described therein does not comprise 5-HTP and *Tanacetum parthenium.*

The present invention falls within the above context, setting out to provide a formulation able both to effectively counteract the symptoms of migraine with aura, and -
when used as a prophylactic treatment - to allow a reduction in the frequency and/or intensity of migraine episodes, and to block the cerebral event underlying the migraine aura if taken immediately at its inception.

The aforesaid objective is achieved by a formulation for use in the treatment of migraine with aura comprising as active ingredients:
i) an extract of Tanacetum parthenium, preferably in a percentage by weight in the range 0.2 -0.45% wt, wherein the extract comprises parthenolide;
ii) magnesium cations, for example in a percentage by weight in the range 0.35-0.55% wt;
iii) 5-hydroxy-tryptophan, advantageously in a percentage by weight in the range 0.1-0.35% wt.

The invention is defined by the claims. Any subject-matter falling outside the scope of the claims is provided for information purposes only.

Preferably, the formulation of the present invention consists of the aforesaid active principles i)-iii).

As regards the component i), Tanacetum parthenium (feverfew) is a perennial herbaceous plant, member of the family of Compositeae. Thanks to the main active components of the extract (preferably dry) it is able to counteract localised stress and promote joint function. In addition, this component provides a prevention mechanism in migraine.

The mechanism of action of feverfew is mediated by the bioactive compound parthenolide, a molecule known for its anti-inflammatory effects which inhibits the biosynthesis of prostaglandins, the release of serotonin biosynthesis of prostaglandins, the release of serotonin by the platelets and also blocks 5HT2A and 5H2b receptors.

The parthenolide interacts with the protein nucleophilic sites by means of an alpha-methylene gamma-lactone ring-and an epoxy portion. This chemical property is characteristic of numerous agonist molecules of the TRPA1 receptor (transient receptor potential ankyrin 1) which, by covalent modification of cysteine residues, determines the activation of ion channels.

The TRPA1 receptor is activated by oxidative/nitrative (NO) stress metabolites generated in inflammation or tissue injury producing pain and mechanical hyperalgesia.

The superfamily of TRP channels represents a diverse and broad group of channel membrane receptors non-selectively permeable to cations.

The subfamily of vanilloid activated receptors or TRPV contains 6 subtypes (1-6), among which TRPV1. TRPV1 and TRPA1 are expressed in primary nociceptive sensory neurons. One of the most important locations of TRPA1 channel is at the level of a sub-population of somatosensitive and nociceptive neurons of the dorsal root ganglia (DRG), trigeminal ganglia or sensory vagal ganglia at the level of these neurons it is co-localised with the TRPV1 receptor channel. Neurons expressing the TRPA1 channel are, in fact, characterised by specific sensitivity to capsaicin, the pungent ingredient in chilli pepper which selectively activates the TRPV1 channel, and for the expression/secretion of pro-inflammatory neuropeptides, the substance P and the calcitonin generelated peptide (CGRP).

In addition to involvement in the transmission of irritant and painful stimuli which trigger reflex responses, the stimulation of TRPA1 positive nerve endings produces a series of local pro-inflammatory responses, due to the release of pro-inflammatory neuropeptides defined overall as "neurogenic inflammation", a key point in the pathophysiology of migraine and migraine aura.

According to an embodiment variant, the extract of the component i) is an extract at 80%.

About the component ii), magnesium is able to adjust the cell membrane potential and permeability of the plasma membrane to different types of ions (potassium, calcium, sodium). This cation contributes to the normal functioning of the nervous system, i.e. to the effective management of the transmission of nerve impulses and to the electrolyte balance. It contributes to normal muscle function. Lastly, being an enzyme cofactor of numerous physiological reactions, it contributes to normal energy metabolism.

Magnesium is an essential cation in many biochemical and neurophysiological processes. It is estimated that at least 325 enzymes are magnesium-dependent and most of these are located in the brain.

An optimal intracellular concentration of magnesium acts as physiological calcium antagonist blocking the "toxic" effects of excessive intracellular calcium, while reduced levels of magnesium favour the formation of free radicals and as a result, toxic concentrations of NO radicals.

Lastly, the component iii) - 5-hydroxy-tryptophan or 5-HTP - is an amino acid precursor of serotonin and an intermediate in the biosynthesis of tryptophan. It is a substance obtained by the transformation of tryptophan in the central nervous system itself .

5-HTP is a precursor of the neurotransmitter serotonin (5-HT) involved in the regulation of mood at the level of the central nervous system. In particular, low levels of 5-HT are associated with various forms of depression. The lack of 5-HT at the central nervous system-level seems also to be associated with anxiety, panic and chronic headache. Serotonin has also proved able to improve sleep quality both in healthy subjects and those with sleeping problems, in particular it is the precursor of melatonin in the pineal gland which acts by improving and increasing REM sleep and deep sleep.

In addition, 5-HTP through its transformation into kynurenic acid (NMDA receptor antagonist), acting as an anti-glutamminergic also comes into play in the treatment of some forms of headache especially migraine demonstrating effectiveness in crisis prevention.

According to an embodiment variant, the component iii) is an extract of griffonia.

According to an embodiment variant, the component iii) extract is an extract at least at 20%, for example 20-98%.

As a result, innovatively, the formulation of the present invention is able to combine the three components listed above to obtain a synergic action in the prevention or symptomatic treatment of migraine with aura.

According to one embodiment, the component i) is in a percentage of about 0.30 -0.40 % wt.

According to a further embodiment, the component ii) is in a percentage of about 0.40 -0.48 % wt.

According to yet a further embodiment, the component iii) is in a percentage of about 0.18 -0.26 % wt.

Preferably the magnesium cations are in oxide form.

According to further variants, that can be implemented independently of each other, the component i) and/or component iii) are in the form of dry extracts, and/or the component i) and/or component iii) are extracted with water, preferably deionised water.

The formulation according to any of the preceding embodiments is also particularly suitable for use in the prophylactic treatment of migraine (particularly migraine aura status) and other forms of headache.

According to further variants, such formulation is characterised by the fact of being in the form of tablets, capsules or sachets, optionally orally dissolving.

Nevertheless the compounds to use, according to the present invention, can be formulated for oral, buccal, transdermal or parenteral administration or in a form suitable for administration by inhalation or insufflation (by mouth or nose).

For oral administration, pharmaceutical compositions may be found, for example, in the form of tablets or capsules prepared in the conventional manner with pharmaceutically acceptable excipients such as binding agents (for example, pregelatinised corn starch, polyvidone or hydroxypropyl methylcellulose); filling agents (such as lactose, microcrystalline cellulose or calcium hydrogen phosphate); lubricants (e.g. magnesium stearate, talc or silica); disintegrating agents (e.g. sodium starch glycolate or potato starch); or inhibiting agents (such as sodium lauryl sulfate). The tablets can be coated using methods well known in the art.

Liquid preparations for oral administration may, for example, be in the form of syrups, solutions or suspensions or be freeze-dried products to be reconstituted before use with water or other appropriate vehicles. These liquid preparations can be prepared using conventional methods with pharmaceutically acceptable additives such as suspension agents (e.g. sorbitol syrup, cellulose derivatives or edible hydrogenated fats); emulsifying agents (for example lecithin or acacia); non-aqueous vehicles (such as almond oil, oily esters, fractionated vegetable oils or ethyl alcohol); and preservatives (such as methyl-or propyl-p-hydroxybenzoate or sorbic acid). The preparation may also suitably contain flavourings, sweeteners, and/or colorants

Preparations for oral administration may be made appropriately to allow the controlled release of active ingredients.

For buccal administration, the compositions may be in the form of tablets or lozenges made in the conventional manner, suitable for absorption at a buccal mucosa level. Typical buccal formulations are tablets for sub-lingual administration.

The compounds according to the present invention can be formulated for parenteral administration by injection. The formulations for the injections may be presented in the form of a single dose, for example in vials, with a preservative added. The compositions may be in such form as suspensions, solutions or emulsions in oily or aqueous vehicles and may contain formulary agents such as suspension agents, stabilizers and/or dispersing agents. Alternatively, the active ingredients may be in the form of powder to be reconstituted before use with an appropriate vehicle, e.g. with sterile water.

In addition to the compositions described above, the compounds can also be formulated as depot preparations. These long-acting formulations can be administered via an implant (for example, transdermal, subcutaneous or intramuscular) or by intramuscular injection. So, for example, the compounds for the use according to the present invention, can be formulated with appropriate hydrophobic or polymer materials (for instance in the form of an emulsion in a suitable oil) or ion-exchange resins or as minimally soluble derivatives, for example as minimally soluble salt.

According to the present invention the dose of the compounds proposed for administration to a man (weighing around 70 kg) ranges from 0.1 mg to 1 g of active ingredients per dosage unit. The dosage unit can be given, for example, from 1 to 4 times a day. The dose depends on the chosen route of administration. Continuous changes to the dosage depending on age and weight of the patient and even the severity of the medical condition being treated may need to be made. The exact dose and route of administration will be at the discretion of the attending physician or veterinarian.

By way of example, in a formulation for an adult patient the components i), ii), iii) could be respectively present in the following weights: 120 mg, 120 mg, 20 mg, or 185 mg, 185 mg, 100 mg.

By way of example, in a formulation for a paediatric patient the components i), ii), iii) could be respectively present in the following weights: 60 mg, 100 mg, 50 mg.

A manufacturing method of a formulation according to any of the preceding embodiments comprises one or more dry mixing steps of the aforementioned components i), ii), iii) .

As regards preferred or additional characteristics of such method, please refer to the description of the formulation above.

Innovatively, the invention described is able to overcome the drawbacks complained of in relation to the prior art.

More precisely, such formulation has proved to be effective in the prophylactic or symptomatic treatment of migraine with aura, without any contraindications being noticeable.

Advantageously, the formulation for the use of the present invention is the first evidence that migraine with aura can be treated effectively at its onset and not, as it has always been done conventionally, by treating a posteriori the symptoms generated by it.

In addition, each variant described as belonging to a possible embodiment may be realised independently of the other embodiments described.

## Claims

1. Formulation for use in the treatment of migraine with aura comprising as active ingredients:
i) an extract of Tanacetum parthenium, in a percentage by weight in the range 0.2 -0.45% wt, wherein the extract comprises parthenolide;
ii) magnesium cations in a percentage by weight in the range 0.35-0.55% wt;
iii) 5-hydroxy-tryptophan in a percentage by weight in the range 0.1 -0.35% wt.

2. Formulation for use according to the previous claim, wherein the component i) is in a percentage by weight of about 0.30 - 0.40 % wt.

3. Formulation for use according to any of the previous claims, wherein the component ii) is in a percentage by weight of about 0.40 - 0.48 % wt.

4. Formulation for use according to any of the preceding claims, wherein the magnesium cations are in the form of oxide.

5. Formulation for use according to any of the previous claims, wherein the component iii) is in a percentage by weight of about 0.18 - 0.26% wt.

6. Formulation for use according to any of the preceding claims, wherein the component i) and/or the component iii) are in the form of dry extracts.

7. Formulation for use according to any of the preceding claims, wherein the component i) and/or the component iii) are extracted with water, preferably deionised water.

8. Formulation for use according to any of the preceding claims, **characterised by** being in the form of tablets, capsules or sachets, optionally lozenges.

9. Formulation for use according to any of the preceding claims for use in the prophylactic treatment of migraine with aura.

## Patentansprüche

1. Formulierung zur Verwendung in der Behandlung von Migräne mit Aura, umfassend als aktive Bestandteile:
i) einen Extrakt von Tanacetum Parthenium in einem Gewichtsprozentanteil in dem Bereich von 0,2 - 0,45 Gew.-%, wobei der Extrakt Parthenolid umfasst,
ii) Magnesiumkationen in einem Gewichtsprozentanteil in dem Bereich von 0,35 - 0,55 Gew.-%,
iii) 5-Hydroxytryptophan in einem Gewichtsprozentanteil in dem Bereich von 0,1 - 0,35 Gew.-%.

2. Formulierung zur Verwendung gemäß dem vorhergehenden Anspruch, wobei die Komponente i) in einem Gewichtsprozentanteil von etwa 0,30 - 0,40 Gew.-% vorliegt.

3. Formulierung zur Verwendung gemäß einem der vorhergehenden Ansprüche, wobei die Komponente ii) in einem Gewichtsprozentanteil von etwa 0,40 - 0,48 Gew.-% vorliegt.

4. Formulierung zur Verwendung gemäß einem der vorhergehenden Ansprüche, wobei die Magnesiumkationen in der Form von Oxid vorliegen.

5. Formulierung zur Verwendung gemäß einem der vorhergehenden Ansprüche, wobei die Komponente iii) in einem Gewichtsprozentanteil von etwa 0,18 - 0,26 Gew.-% vorliegt.

6. Formulierung zur Verwendung gemäß einem der vorhergehenden Ansprüche, wobei die Komponente i) und/oder die Komponente iii) in der Form von Trockenextrakten vorliegen.

7. Formulierung zur Verwendung gemäß einem der vorhergehenden Ansprüche, wobei die Komponente i) und/oder die Komponente iii) mit Wasser, vorzugsweise entionisiertem Wasser, extrahiert sind.

8. Formulierung zur Verwendung gemäß einem der vorhergehenden Ansprüche, **gekennzeichnet durch** das Vorliegen in der Form von Tabletten, Kapseln oder Sachets, vorzugsweise Lutschtabletten.

9. Formulierung zur Verwendung gemäß einem der vorhergehenden Ansprüche zur Verwendung in der prophylaktischen Behandlung von Migräne mit Aura.

## Revendications

1. Formulation pour une utilisation dans le traitement de la migraine avec aura comprenant comme ingrédients actifs :
i) un extrait de Tanacetum parthenium, à un pourcentage en poids dans la plage de 0,2 à 0,45 % en poids, dans laquelle l'extrait comprend un parthénolide ;
ii) des cations de magnésium à un pourcentage en poids dans la plage de 0,35 à 0,55 % en poids ;
iii) du 5-hydroxy-tryptophane à un pourcentage en poids dans la plage de 0,1 à 0,35 % en poids.

2. Formulation pour une utilisation selon la revendication précédente, dans laquelle le composant i) est à un pourcentage en poids d'environ 0,30 à 0,40 % en poids.

3. Formulation pour une utilisation selon l'une quelconque des revendications précédentes, dans laquelle le composant ii) est à un pourcentage en poids d'environ 0,40 à 0,48 % en poids.

4. Formulation pour une utilisation selon l'une quelconque des revendications précédentes, dans laquelle les cations de magnésium sont sous forme d'oxyde.

5. Formulation pour une utilisation selon l'une quelconque des revendications précédentes, dans laquelle le composant iii) est à un pourcentage en poids d'environ 0,18 à 0,26 % en poids.

6. Formulation pour une utilisation selon l'une quelconque des revendications précédentes, dans laquelle le composant i) et/ou le composant iii) sont sous la forme d'extraits secs.

7. Formulation pour une utilisation selon l'une quelconque des revendications précédentes, dans laquelle le composant i) et/ou le composant iii) sont extraits avec de l'eau, de préférence de l'eau désionisée.

8. Formulation pour une utilisation selon l'une quelconque des revendications précédentes, **caractérisée en ce qu'**elle se présente sous forme de comprimés, de capsules ou de sachets, facultativement de pastilles.

9. Formulation pour une utilisation selon l'une quelconque des revendications précédentes pour une utilisation dans le traitement prophylactique de la migraine avec aura.
